# FASCICULE DE BREVET EUROPEEN

(11) **EP 0 956 062 B1**
(45) Date de publication et mention de la délivrance du brevet: **26.03.2003**
(21) Numéro de dépôt: 98956970.2
(22) Date de dépôt: 27.11.1998
(51) Int. Cl.: A61M 5/20

(54) **INJECTEUR MEDICAL**
MEDIZINISCHER INJEKTOR
INJECTOR FOR MEDICAL USE

(30) Priorité: 03.12.1997 CA 2223659
(43) Date de publication de la demande: 17.11.1999
(73) Titulaire: Villette, Alain, 79700 Saint Pierre des Echaubrognes (FR)
(72) Inventeur: Villette, Alain, 79700 Saint Pierre des Echaubrognes (FR)
(74) Mandataire: Viard, Jean
(86) Numéro de dépôt international: FR9802551
(87) Numéro de publication internationale: WO99027984

(56) Documents cités:
- WO-A-92/08410
- FR-A- 2 418 652
- FR-A- 2 581 548
- FR-A- 2 716 375
- GB-A- 2 067 076
- US-A- 5 290 261

## Description

La présente invention a pour objet un injecteur destiné à un usage en dentisterie, pour réaliser des injections intramédullaires ou intradiploïques d'anesthésique ou des injections dans les tissus mous.

En chirurgie dentaire, la solution classique consiste à injecter à l'aide d'une seringue une quantité déterminée d'anesthésique liquide dans la gencive du patient à une certaine distance de la dent à soigner. Mais cette procédure présente des inconvénients tels qu'un temps de latence important entre l'injection et l'action anesthésiante, et l'injection d'une quantité d'anesthésique plus importante que nécessaire. Pour remédier à ces inconvénients, il a déjà été proposé de réaliser des anesthésies intradiploïques dans lesquelles l'injection est effectuée directement dans la moelle de l'os maxillaire, après traversée de la corticale. L'action de l'anesthésique est ainsi plus efficace. A cet effet, il est connu de faire tourner l'aiguille d'injection pour faciliter la pénétration de l'aiguille dans la corticale qui est une région dure. Après quoi, un piston est déplacé qui porte contre le fond mobile de la carpule contenant l'anesthésique.

Il a déjà été proposé de réaliser, notamment dans US-A-5,173,050, une telle anesthésie en deux temps. On procède dans un premier temps au perçage de la corticale, après quoi l'injecteur est retiré, et l'aiguille de perforation remplacée par une aiguille d'injection. Mais il est très difficile de retrouver la perforation pour procéder à l'injection.

Un injecteur permettant de réaliser une telle anesthésie sans avoir à dégager l'aiguille de perforation est décrit dans FR-A-2 581 548. Dans ce brevet, les moyens d'entraînement en rotation de la carpule et de translation du fond mobile de la carpule sont constitués par deux micromoteurs, la liaison mécanique entre le moteur d'entraînement en rotation de la carpule et celle-ci étant obtenu par l'intermédiaire d'engrenages. Il en va de même de l'entraînement du piston que est obtenu par un engrenage réducteur. Il est ainsi possible d'obtenir une injection continue et régulière ce qui n'était pas le cas avec les seringues à main. Mais il est apparu que ces entraînements généraient des bruits indésirables, le moteur tournant à des vitesses très élevées de plusieurs dizaines de milliers de tours par minute.

La présente invention a pour objet de pallier ces inconvénients. Elle est basée sur l'idée que tous les efforts à transmettre doivent l'être dans l'axe de l'injecteur en évitant tout renvoi mécanique qui se traduit assez fréquemment par une excentricité des efforts appliqués entraînant des bris de carpule.

Un injecteur comprenant les caractéristiques du préambule de la revendication 1 est connu du document FR-A-2 716 375.

Selon l'invention, l'injecteur comprend les caractéristiques de la revendication 1.

De préférence, la vis d'entraînement du moteur présente un moyeu traversé par des vis de fixation.

L'effort appliqué sur le fond de la carpule ou container est parfaitement coaxial à celle-ci et l'entraînement en translation se fait sans glissement.

Selon une autre caractéristique de l'invention, la commande du moteur d'injection est programmée de sorte que le volume injecté soit progressif, ce qui diminue très sensiblement la douleur.

D'autres caractéristiques et avantages de l'invention apparaîtront au cours de la description qui va suivre de modes particuliers de réalisation, donnés uniquement à titre d'exemples non limitatifs, en regard des dessins qui représentent :
- La Figure 1, une vue en coupe d'un injecteur dentaire selon l'invention;
- La Figure 2, un vue de détail du montage du moteur d'injection;
- La figure 3, une coupe selon la ligne III-III de la figure 2;
- La Figure 4, une vue en coupe partielle du montage de la carpule dans un injecteur d'anesthésie dentaire.
- La Figure 5, un schéma de l'étage électronique de commande de l'injecteur;
- La Figure 6, un graphique représentant la variation de la vitesse d'injection en fonction du temps en vue de rendre l'injection indolore.

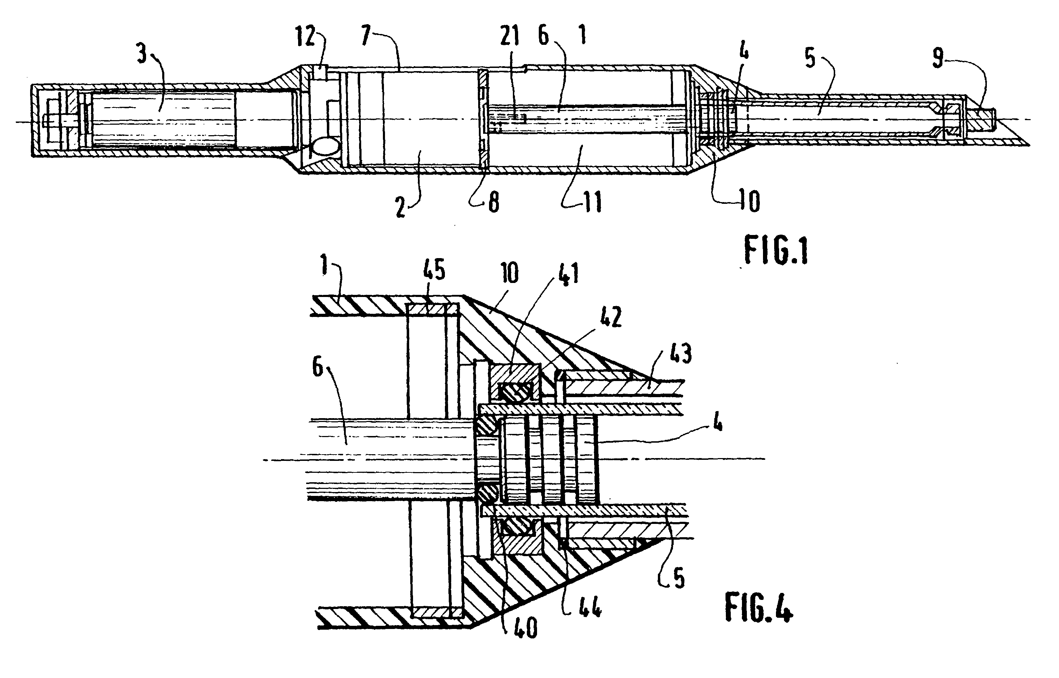

La figure 1 représente un injecteur destiné à l'anesthésie dentaire de type intradiploïque c'est-à-dire impliquant la pénétration de l'aiguille dans un os avant injection. Dans ce cas, il est nécessaire de prévoir, outre le moteur de translation ou de déplacement axial servant à injecter l'anesthésique, un moteur supplémentaire susceptible de communiquer à l'aiguille un mouvement de rotation facilitant la traversée de l'os avant l'injection proprement dite.

Sur la figure 1, on distingue, dans un corps cylindrique creux 1, un moteur 2 d'entraînement en rotation de la carpule 5 et de l'aiguille 9 et un moteur 3 de déplacement axial du moteur 2, d'un piston 6 dont l'extrémité avant porte contre le fond 4 coulissant de la carpule 5. Sur l'avant du corps 1 est monté un logement partiellement tronconique 10 de maintien et de guidage de la carpule 5.

Conformément à l'invention, le moteur de rotation 2 est coaxial au moteur d'injection 3, à la carpule 5 ou réservoir d'anesthésique et à l'aiguille 9. Comme cela apparaît sur la figure 1, le moteur 2 est disposé à l'avant du moteur d'injection 3 dans la cage 11 et son arbre de sortie est mécaniquement relié au piston 6. A l'extrémité avant de l'injecteur est fixée dans un logement 10 prévu à cet effet une carpule 5 et, sur l'avant de la carpule 5, est fixée une aiguille 9 dont l'extrémité arrière traverse le joint avant de la carpule pour établir une communication entre la carpule 5 et le canal intérieur de l'aiguille 9. Le logement 10 forme un palier pour la carpule et par suite, l'aiguille. Le moteur 2, lorsqu'il est alimenté entraîne ainsi normalement l'aiguille 9 en rotation ce qui facilite la perforation de l'os. Dans un premier temps, il reste immobile, la pression nécessaire provenant de la pression exercée par le chirurgien-dentiste.

Dans un second temps, la mise en rotation de l'arbre 31 du moteur 3 provoque un déplacement de la position de repos, à gauche sur la figure 1, vers la droite.

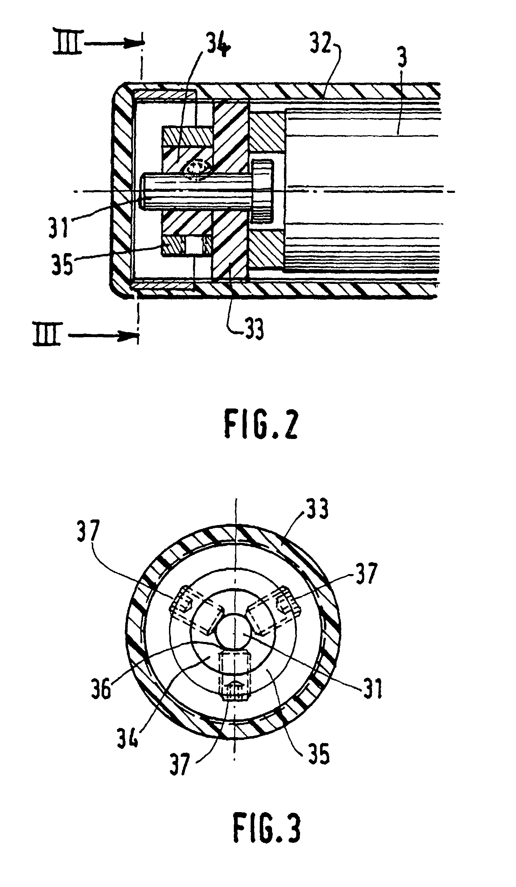

Comme cela apparaît plus clairement sur la figure 2 qui est une vue de détail à échelle agrandie de la partie arrière de l'injecteur et sur la figure 3, à l'intérieur du corps 1 de l'injecteur, et dans la partie arrière de celui-ci est monté un fourreau ou manchon 32 qui est fileté intérieurement. Une vis 33 en forme de disque, avantageusement en « DELRIN » (Marque déposée), est filetée extérieurement au même pas que le filetage interne du manchon 32 et est susceptible de se déplacer dans celui-ci lorsqu'elle est entraînée en rotation. La vis 32 se prolonge vers l'arrière par un moyeu 34. Ce moyeu est entouré par une couronne métallique 35 L'entraînement en rotation de la vis 33 résulte d'une liaison mécanique entre l'arbre moteur 31 et le moyeu 34 de la vis 33. A cet effet, l'arbre lisse 31 présente un méplat 36. Sur ce méplat, prend appui une vis radiale 37 qui traverse la couronne métallique 35 et le moyeu 34 pour venir en contact avec l'arbre moteur 31. Mais étant donné que l'effort à transmettre est important, on prévoit, de préférence, deux autres vis radiales 37 chacune formant un angle de 120° avec les vis adjacentes. Le couple provenant de la rotation de l'arbre moteur 31 est ainsi transmis, sans risque de rupture à la vis 33.

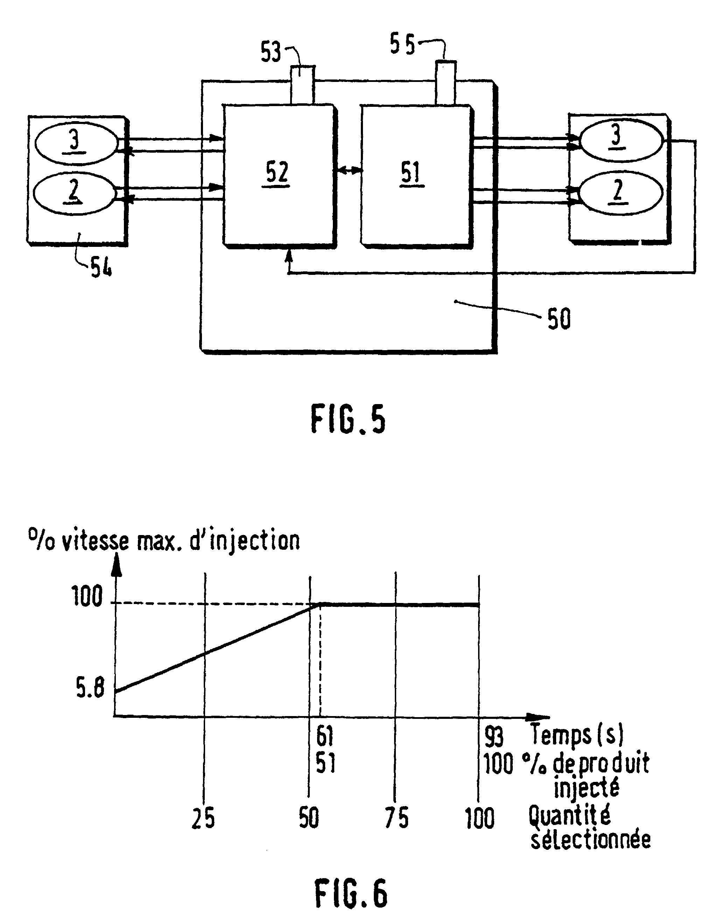

On comprendra aisément que l'avance et/ou le recul du moteur 3 à l'intérieur du fourreau 32 dépendra du sens de rotation de l'arbre moteur, lui-même dépendant du sens du courant continu d'alimentation qui est commandé par une carte électronique 50 (fig.5).

La figure 4 représente à échelle agrandie et en coupe le montage de la carpule dans l'embout 10. On retrouve sur cette figure l'extrémité avant du piston 6, la carpule 5 fermée à sa partie arrière par un disque souple 4, par exemple en néoprène, portant sur la surface intérieure de la carpule 5 pour assurer l'étanchéité de celle-ci. L'embout 10 est, par exemple vissé sur le corps 1 de l'injecteur et constitue un logement pour le porte-aiguille 43. le piston 6 porte à sa partie avant destinée à pénétrer dans la carpule 5 un joint torique 40. L'arrière de la carpule 5 est maintenu dans un palier 41 par un second joint torique 42 qui est écrasé pour guider efficacement l'arrière de la carpule lorsqu'elle est entraînée en rotation sous l'action du moteur 2.

Le fonctionnement d'un tel injecteur est le suivant : Dans un premier temps, sous la pression appliquée par le chirurgien-dentiste, la muqueuse est traversée par l'aiguille 9 dont l'extrémité avant arrive au contact de l'os. Le circuit de commande permet dans cette position l'alimentation du moteur 2 et la mise en rotation de son arbre moteur et, par suite du piston 6, de la carpule 5 et de l'aiguille 9. L'os est alors traversé et l'extrémité avant de l'aiguille arrive au contact de la moelle. Le moteur 2 est alors stoppé et le moteur 3 est alimenté ce qui provoque l'injection de l'anesthésique contenu dans la carpule. Après quoi, le sens de rotation du moteur 2 est inversé et l'aiguille 9 est retirée de la mâchoire.

Il est ainsi possible de disposer d'une poussée axiale régulée par le déplacement du moteur 3 tournant à faible vitesse. Le déplacement de la vis 12 dans la rainure 7, empêche toute rotation de la cage 3.

La figure 5 est un schéma du circuit électronique de commande de l'injecteur. Il comprend essentiellement une carte électronique 50 incluant un étage de commande 51 et un étage de contrôle ou de surveillance 52. La carte électronique a pour fonction de gérer le fonctionnement des moteurs à courant continu 2 et 3, les deux moteurs tournant toujours dans le même sens. Le sens de rotation est géré soit automatiquement en fonction de la position et du couple du moteur d'injection 3, soit manuellement par appui sur la touche retour. Le courant consommé par le moteur d'injection est mesuré en permanence. Ainsi, si au cours de l'injection, le couple atteint une valeur prédéterminée, par exemple 40 daN, qui correspond à une injection dans un os dur, le circuit de commande 51 inverse immédiatement le sens du courant pendant deux secondes.

A la mise sous tension de la carte, le moteur d'injection est ramené automatiquement en position reculée et ensuite sa position est connue à tout moment par le calcul effectué par un microprocesseur. L'injecteur est protégé par un contact de fin de course (non représenté). Dans la phase finale de l'injection, quand 90% de l'anesthésique est injecté, une augmentation du courant consommé entraîne l'arrêt du moteur 3 et son retour en position initiale. Dans la phase de retour en position initiale, le moteur est arrêté dès qu'une surconsommation est détectée.

Le chirurgien dispose d'une commande d'injection; d'une commande de rotation; d'un interrupteur MARCHE/ARRÊT; d'un bouton poussoir 53 pour sélectionner la quantité de produit à injecter et d'un bouton de rappel manuel 55 du moteur 3 dans sa position initiale. De préférence, la commande des moteurs est effectuée au pied par l'intermédiaire d'une pédale double 54, connectée à l'étage de contrôle 52, présentant deux surfaces d'appui dont l'une permet de commander le fonctionnement du moteur de rotation 2 et dont l'autre permet de commander l'injection par l'intermédiaire du moteur 3.

La touche 53 permet la sélection préalable de la quantité de produit à injecter, c'est à dire du déplacement du moteur d'injection 3. un appui correspond à 25% de la quantité totale, deux appuis 50%, trois appuis 75% quatre appuis 100%. Le choix de la commande est indiqué par des diodes luminescentes (non représentées). Comme indiqué précédemment, il est souhaitable que la vitesse d'injection soit progressive ce qui implique que le déplacement linéaire du moteur 3 soit un déplacement accéléré.

La figure 6 est un graphique sur lequel a été indiqué en abscisse le temps et en ordonnées la vitesse d'injection qui varie de 0 à 100%. On a constaté que la douleur provoquée par l'injection d'anesthésique provenait essentiellement de ce que l'injection se faisait brutalement après perforation de la corticale. Grâce à la présence de la carte électronique 50, il est possible de réguler l'injection de sorte que celle-ci soit progressive et, par suite non douloureuse. Comme cela apparaît sur le graphique, la vitesse d'injection commence à 5,8% pour atteindre 100%, soit la vitesse maximale d'injection, lorsqu'environ 50% de l'anesthésique a été injecté. L'échelle inférieure graduée de 25 à 100 symbolise la quantité d'anesthésique à injecter qui est sélectionnée par le bouton 53.

L'injecteur selon l'invention peut également être utilisé pour l'injection dans des tissus mous qui sont parcourus par des vaisseaux de diamètres plus ou moins importants. Il est alors nécessaire de vérifier que l'extrémité de l'aiguille ne se trouve pas dans une artère ou une veine. Tel est le cas, en dentisterie, lorsque l'on réalise une anesthésie dans la mandibule au voisinage de l'épine de SPIX. L'injecteur doit alors offrir la possibilité d'une aspiration.

Pour obtenir cette vérification, l'extrémité du tube porte-cartouche 43 est réalisée en une matière plastique transparente telle que du polycarbonate (stérilisable à 130°C). Parallèlement, le microprocesseur est programmé pour permettre, après deux appuis successifs sur la touche 53 de « quantité » un retour du piston, alors que dans le mode de réalisation précédent seul un retour automatique était possible. Le joint torique 40 situé à l'extrémité avant du piston 6 entraînant la carpule en rotation crée une étanchéité entre le piston en caoutchouc 4 de la cartouche d'anesthésique et le piston tournant 6 de manière à solidariser les deux pistons. Si le piston 6 recule, il aspire le piston 4 de la cartouche et crée à l'intérieur de celle-ci qui se traduit par un afflux de sang si l'extrémité de l'aiguille se trouve dans un vaisseau. Cet afflux peut être constaté grâce à la transparence du tube porte cartouche.
Il va de soi que de nombreuses variantes peuvent être apportées, notamment par substitution de moyens techniques équivalents, sans sortir pour cela du cadre de l'invention.

## Revendications

1. Injecteur comprenant un moteur (2) entraînant un piston (6) en rotation et un second moteur (3) entraînant ledit piston (6) en translation, le piston étant en contact avec le fond mobile d'une carpule (5) fixée d'une manière amovible sur le corps (1) de l'injecteur, dans lequel le moteur d'entraînement (3) en translation du piston (6) est coaxial à celui-ci et monté à l'intérieur du corps cylindrique (1) de l'injecteur, son entraînement en translation résultant de l'entraînement par l'arbre moteur d'une vis (33) coopérant avec un filetage (32) intérieur au corps de l'injecteur, **caractérisé en ce que** le fonctionnement des moteurs (2, 3) est commandé par une carte électronique programmable (50).

2. Injecteur selon la revendication 1, dans lequel, la carte électronique (50) comprend un étage de commande (51), un étage de contrôle (52), un bouton de sélection (53), un interrupteur (55) et un jeu (54) de deux pédales commandant respectivement les fonctionnements des moteurs de rotation (2) et d'injection (3).

3. Injecteur selon l'une des revendications 1 ou 2 dans lequel, l'arbre moteur (31) du moteur (3) est solidaire par l'intermédiaire d'un moyeu (34) d'au moins une vis (33) dont le filetage extérieur coopère avec le filetage interne d'un manchon (32) fixe à l'intérieur du corps (1).

4. Injecteur selon la revendication 1 dans lequel le moteur d'injection (3) est mobile en translation à l'intérieur du corps (1) et destiné à pousser une paroi mobile de la carpule (5), portée par un tube porte-carpule, à l'aide du piston (6) et le moteur de rotation (2) pouvant entraîner en rotation la carpule (5) et l'aiguille (9) montée sur la carpule (5) en vue de faciliter la pénétration de l'aiguille.

5. Injecteur selon l'une quelconque des revendications précédentes dans lequel le moteur d'injection (3) est maintenu en position par une vis (12) incluse dans une rainure (7).

6. Injecteur selon la revendication 1 **caractérisé en ce que** le microprocesseur est programmé pour permettre, après deux appuis successifs sur la touche (53) un retour du piston.

7. Injecteur selon la revendication 4 dans lequel l'extrémité du tube porte-carpule (43) est réalisée en une matière plastique transparente.

## Claims

1. Injector comprising a motor (2) driving a plunger (6) in rotation and a second motor (3) driving the said plunger (6) in translation, the plunger being in contact with the movable bottom of a cartridge (5) detachably fastened to the body (1) of the injector, in which the motor (3) driving the plunger (6) in translation is coaxial therewith and mounted inside the cylindrical body (1) of the injector, its translational drive resulting from the motor shaft driving a screw (33) cooperating with a thread (32) inside the body of the injector, **characterized in that** the operation of the motors (2, 3) is controlled by a programmable electronic card (50).

2. Injector according to Claim 1, in which the electronic card (50) comprises a control stage (51), a monitoring stage (52), a selection button (53), a switch (55) and a set (54) of two pedals respectively controlling the operation of the rotation (2) and injection (3) motors.

3. Injector according to either of Claims 1 and 2, in which the motor shaft (31) of the motor (3) is secured via a hub (34) of at least one screw (33), the external thread of which cooperates with the internal thread of a sleeve (32) fastened inside the body (1).

4. Injector according to Claim 1, in which the injection motor (3) can be moved in translation inside the body (1) and is intended to push a movable wall of the cartridge (5), carried by a cartridge-carrier tube, by means of the plunger (6) and the rotary motor (2) which can drive the cartridge (5) and the needle (9) mounted on the cartridge (5) in rotation for the purpose of facilitating the penetration of the needle.

5. Injector according to any one of the preceding claims, in which the injection motor (3) is held in position by a screw (12) inserted into a groove (7).

6. Injector according to Claim 1, **characterized in that** the microprocessor is programmed in order to allow the plunger to return after the button (53) is pressed twice in succession.

7. Injector according to Claim 4, in which the end of the cartridge-carrier tube (43) is made from a transparent plastic.

## Patentansprüche

1. Injektor umfassend einen Motor (2), der einen Kolben (6) in Rotation antreibt und einen zweiten Motor (3), der den Kolben in Translation antreibt, wobei der Kolben mit dem mobilen Boden einer unbeweglich auf dem Körper (1) des Injektors befestigten Spritze in Kontakt steht, in dem der Translationsantriebsmotor (3) des Kolbens (6) koaxial dazu ist und im Inneren des zylindrischen Körpers (1) des Injektors angebracht ist, wobei sein Translationsantrieb aus dem Antrieb einer Schraube (33) durch die Motorwelle erfolgt, die mit einem Innengewinde (32) im Körper des Injektors kooperiert, **dadurch gekennzeichnet, dass** die Funktion der Motoren (2, 3) durch eine programmierbare elektronische Schaltung (50) gesteuert wird.

2. Injektor nach Anspruch 1, bei dem die elektronische Schaltung (50) eine Steuerungsstufe (51), eine Kontrollstufe (52), einen Auswahlknopf (53) , einen Unterbrecher (55) und eine Anordnung (54) von zwei Pedalen umfasst, die entsprechend die Funktionen der Motoren zur Rotation (2) und Injektion (3) steuern.

3. Injektor nach einem der Ansprüche 1 oder 2, bei dem die Motorwelle (31) des Motors (3) mittels einer Nabe (34) mit mindestens einer Schraube (33) verbunden ist, deren Außengewinde mit dem Innengewinde einer Muffe (32) kooperiert, die im Inneren des Körpers (1) befestigt ist.

4. Injektor nach Anspruch 1, bei dem der Injektionsmotor (3) im Inneren des Körpers (1) in Translation beweglich ist und vorgesehen ist, mit Hilfe eines Kolbens (6) eine bewegliche Trennwand der Spritze (5) zu schieben, die durch ein Spritzenträgerrohr getragen ist, und wobei der Rotationsmotor (2) die Spritze (5) und die auf der Spritze (5) angebrachte Nadel (9) in Rotation antreiben kann, um das Eindringen der Nadel zu erleichtern.

5. Injektor nach einem der vorhergehenden Ansprüche, bei dem der Injektionsmotor (3) durch eine Schraube (12) in Position gehalten ist, die in einer Nut (7) vorgesehen ist.

6. Injektor nach Anspruch 1, **dadurch gekennzeichnet, dass** der Mikroprozessor so programmiert ist, dass er nach zweimal aufeinanderfolgendem Drücken auf die Taste (53) eine Rückführung des Kolbens ermöglicht.

7. Injektor nach Anspruch 4, bei dem das Ende des Spritzenträgerrohrs (43) aus einem transparenten Kunststoffmaterial ausgebildet ist.
